# EUROPEAN PATENT APPLICATION

(11) **EP 4 403 229 A2**
(43) Date of publication of application: **24.07.2024**
(21) Application number: 24176375.4
(22) Date of filing: 31.07.2017
(51) Int. Cl.: A61P 35/00

(54) **EXPRESSION OF PTEN-LONG WITH ONCOLYTIC VIRUSES**

(30) Priority: 29.07.2016 US 201662368822 P; 31.03.2017 US 201762479671 P
(62) Divisional of application: 17835414.8
(71) Applicant: Ohio State Innovation Foundation, Columbus, OH 43201 (US)
(72) Inventor: KAUR, Balveen, Houston, TX 77030 (US); RUSSELL, Luke, Rochester, MN 55906 (US)
(74) Representative: The IP Asset Partnership Limited

(57) **Abstract**

Disclosed are novel modified viruses comprising recombinant PTEN-Long and methods of using the same for treating cancer.

## Description

### I. STATEMENT OF GOVERNMENT SUPPORT

1. This work was supported by NIH 1R01NS064607 and NIH P01 CA163205 awarded by the National Institutes of Health. The government has certain rights in the invention.

### II. CROSS REFERENCE TO RELATED APPLICATIONS

2. This application claims the benefit of U.S. Provisional Patent Application Serial No. 62/368,822, filed July 29, 2016, and U.S. Provisional Patent Application Serial No.62/479,671, filed March 31, 2017, each of which is expressly incorporated herein by reference in its entirety.

### III. BACKGROUND

3. Glioblastoma (GBM) is a World Health Organization grade IV astrocytoma with poor patient survival, characterized by extreme heterogeneity and invasiveness, and by resistance to radiation and chemotherapeutic drugs. Brain metastases are 2-3x more frequent than GBM, with breast cancer among the top 3 brain metastasizing tumor types. Median survival of patients receiving the current standard of care is under 18 months for GBM and 2-25 months for breast cancer brain metastases (BCBM). What are needed are novel therapies for brain tumors and cancers in general.

4. Glioblastoma (GBM) is a World Health Organization grade IV astrocytoma with poor patient survival, characterized by extreme heterogeneity and invasiveness, and by resistance to radiation and chemotherapeutic drugs. Brain metastases are 2-3x more frequent than GBM, with breast cancer among the top 3 brain metastasizing tumor types. Median survival of patients receiving the current standard of care is under 18 months for GBM and 2-25 months for breast cancer brain metastases (BCBM). What are needed are novel therapies for brain tumors and cancers in general.

### IV. SUMMARY

5. Disclosed are methods and compositions related to recombinant viruses expressing a mammalian PTEN-Long gene.

6. In one aspect disclosed herein are recombinant viruses of any preceding aspect, wherein the recombinant virus is a recombinant HSV-1 virus comprising a PTEN-Long gene.

7. In one aspect, disclosed herein are recombinant viruses of any preceding aspect, wherein the virus has been modified to target tumor cells or modified to place genes critical to viral replication under the control of a tumor-specific promoter.

8. In one aspect, disclosed herein are recombinant viruses of any preceding aspect, wherein the PTEN-Long gene is operatively linked to a tissue specific promoter, inducible promoter, or a constitutive promoter.

9. Also disclosed herein are methods of treating a cancer comprising administering to a subject the recombinant virus of any preceding aspect.

10. In one aspect, disclosed herein are methods of inhibiting a cancer and/or inhibiting metastasis comprising administering to a subject the recombinant virus of any preceding aspect.

11. Also disclosed herein are methods inhibiting PD-L1 in a cancer cell comprising administering to the subject the virus of any preceding aspect.

12. In one aspect, disclosed herein are methods of increasing the number of infiltrating CD8 T cells at the site of a cancer in a subject, comprising administering to the subject the virus of any preceding aspect.

13. Also disclosed are methods of increasing the number of infiltrating Natural Killer cells at the site of a cancer in a subject, comprising administering to the subject the virus of any preceding aspect.

### V. BRIEF DESCRIPTION OF THE DRAWINGS

14. The accompanying drawings, which are incorporated in and constitute a part of this specification, illustrate several embodiments and together with the description illustrate the disclosed compositions and methods.

15. Figure 1A shows a PTEN-Long transgene expression vector within HSVQuik backbone.

16. Figure 1B shows transgene expression is detectable within 3 hours of infection in U87ΔEGFR glioma cells (WB).

17. Figure 1C shows that HSV-P10 infection dramatically affects Akt phosphorylation in infected DB7 cells compared with HSVQ control (WB).

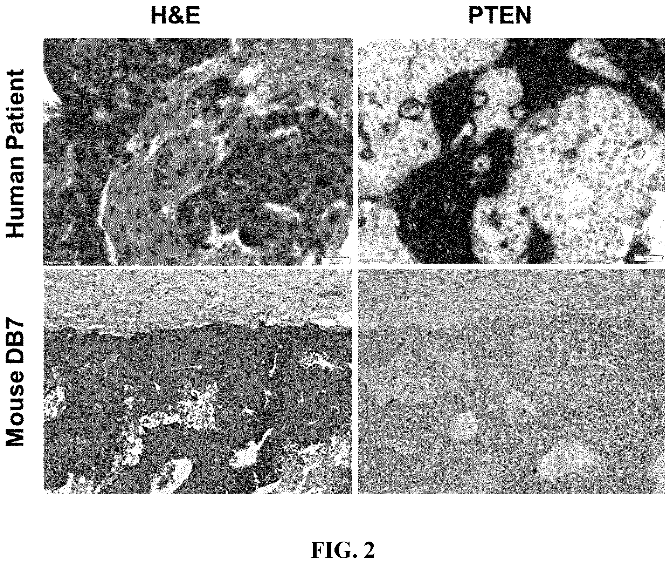

18. Figure 2 shows representative hematoxylin and eosin (H&E) and PTEN staining from a sectioned human breast cancer brain metastasis (top, 40X) and FVB/N mouse bearing intracranial DB7 (bottom, 20X).

19. Figure 3 shows that HSV comprising a PTEN-Long expression vector (HSV-P10) can replicate and spread in glioma cells in vitro as well as control HSVQ virus.

20. Figures 4A, 4B, 4C, and 4D show that PTEN-Long expression does not inhibit HSV-P10 cell killing in multiple cell lines. MTT assays (DB7, murine breast cancer, MDA-MB-468, human breast cancer, LN229 and U87ΔEGFR, human glioma).

21. Figure 5A shows the survival of DB7-dsRed tumor bearing FVB/N mice. Virus injected intratumorally 7d post tumor implantation.

22. Figure 5B shows the survival of DB7 tumor bearing NUDE mice. Virus injected intratumorally 7d post tumor implantation.

23. Figures 6A, 6B, 6C, and 6D show brain immune infiltrates 7 days post-virus treatment. Figure 6A shows the ratio of CD11b⁺ F4/80⁺ microglia (CD45^{lo-int}) to macrophages (CD45^{hi}). Figure 6B shows the percentage of MHC-II⁺ microglia (red) and macrophages (green). Figure 6C shows infiltrating CD49b⁺ CD335 ⁺ NK cells. Figure 6D shows infiltrating cytotoxic CD3 ⁺ CD8 ⁺ T-cells.

24. Figure 7 shows that HSV-P10 abrogates virally induced PD-L1 expression on the surface of infected and neighboring uninfected DB7 breast cancer cells 24hpi. Measured by flow cytometry.

25. Figure 8 shows the serum from tumor-bearing FVB/N mice harvested 7 days post treatment, analyzed for antibodies against DB7 cell lysate. n=3 per group. Measured by ELISA.

26. Figure 9 shows a representation of the pathways in which PTEN-Long expressing recombinant viruses (such as HSV-P10 also known as RAPTOR) can attack cancer cells. As shown in the figures anti-tumor antibody responses increase, and due to the decreased phosphorylation of AKT, PD-L1 expression is decreased preventing inhibitory signals that reduce T cell and NK cell proliferation. Therefore, infiltrating NK cells and T cells increase and can exert anti-tumor activity.

### VI. DETAILED DESCRIPTION

27. Before the present compounds, compositions, articles, devices, and/or methods are disclosed and described, it is to be understood that they are not limited to specific synthetic methods or specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

### A. Definitions

28. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

29. Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10"as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

30. In this specification and in the claims which follow, reference will be made to a number of terms which shall be defined to have the following meanings:

31. "Optional" or "optionally" means that the subsequently described event or circumstance may or may not occur, and that the description includes instances where said event or circumstance occurs and instances where it does not.

32. A "decrease" can refer to any change that results in a smaller amount of a symptom, composition, or activity. A substance is also understood to decrease the genetic output of a gene when the genetic output of the gene product with the substance is less relative to the output of the gene product without the substance. Also for example, a decrease can be a change in the symptoms of a disorder such that the symptoms are less than previously observed.

33. "Inhibit," "inhibiting," and "inhibition" mean to decrease an activity, response, condition, disease, or other biological parameter. This can include but is not limited to the complete ablation of the activity, response, condition, or disease. This may also include, for example, a 10% reduction in the activity, response, condition, or disease as compared to the native or control level. Thus, the reduction can be a 10, 20, 30, 40, 50, 60, 70, 80, 90, 100%, or any amount of reduction in between as compared to native or control levels.

34. As used herein, "treatment," "treat," or "treating" can mean a method of reducing the effects of a disease or condition. Treatment can also refer to a method of reducing the disease or condition itself rather than just the symptoms. The treatment can be any reduction from native levels and can be but is not limited to the complete ablation of the disease, condition, or the symptoms of the disease or condition. Therefore, in the disclosed methods, "treatment" can refer to a 10%, 20%, 30%, 40%, 50%, 60%, 70%, 80%, 90%, or 100% reduction in the severity of an established disease or the disease progression. For example, a disclosed method for reducing, or inhibiting the effects of a cancer (such as inhibiting or reducing metastasis) is considered to be a treatment if there is a 10% reduction in one or more symptoms of the disease in a subject with the disease when compared to native levels in the same subject or control subjects. Thus, the reduction can be a 10, 20, 30, 40, 50, 60, 70, 80, 90, 100%, or any amount of reduction in between as compared to native or control levels. It is understood and herein contemplated that "treatment" does not necessarily refer to a cure of the disease or condition, but an improvement in the outlook of a disease or condition.

35. Throughout this application, various publications are referenced. The disclosures of these publications in their entireties are hereby incorporated by reference into this application in order to more fully describe the state of the art to which this pertains. The references disclosed are also individually and specifically incorporated by reference herein for the material contained in them that is discussed in the sentence in which the reference is relied upon.

### B. Compositions

36. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular PTEN-Long expressing recombinant virus is disclosed and discussed and a number of modifications that can be made to a number of molecules including the PTEN-Long and/or the viral backbone are discussed, specifically contemplated is each and every combination and permutation of PTEN-Long and any viral backbone and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

37. The tumor suppressor phosphatase and tensin homolog on chromosome ten (PTEN) is a phosphatase which inhibits the AKT signaling pathway in cells. PTEN is mutated or deleted in ~50% of all high grade gliomas and is lost in 33-49% of all breast cancers. PTEN-Long is a recently discovered alternative start site variant of PTEN which is has 173 additional amino acids at the N-terminus. This longer form of PTEN is secreted and membrane permeable, and retains its phosphatase activity after secretion and re-entry into cells. Disclosed herein are novel tumor therapies (for example, brain tumor therapies) using a modified virus (such as, for example a modified herpes simplex virus (HSV-P10)) which secretes PTEN-Long from infected cells.

### 1. Delivery of the compositions to cells

38. In one aspect, it is understood and contemplated herein that the functional and therapeutic effects disclosed herein are a result of the expression of PTEN-Long in cells, such as for example, cancer cells. There are a number of compositions and methods which can be used to deliver nucleic acids (such as PTEN-Long expressing nucleic acids) to cells, either *in vitro* or *in vivo.* These methods and compositions can largely be broken down into two classes: viral based delivery systems and non-viral based delivery systems. For example, the nucleic acids can be delivered through a number of direct delivery systems such as, electroporation, lipofection, calcium phosphate precipitation, plasmids, viral vectors, viral nucleic acids, phage nucleic acids, phages, cosmids, or via transfer of genetic material in cells or carriers such as cationic liposomes. Accordingly, in one aspect are viral vectors, plasmids, phages, cosmids, comprising PTEN-Long. Also disclosed herein are methods of treating cancer in a subject comprising administering to the subject PTEN-Long wherein the PTEN-Long is delivered to the cancer cells via lipofection, calcium phosphate precipitation, plasmids, viral vectors, viral nucleic acids, phage nucleic acids, phages, cosmids, or via transfer of genetic material in cells or carriers such as cationic liposomes. Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991). Such methods are well known in the art and readily adaptable for use with the compositions and methods described herein. In certain cases, the methods will be modified to specifically function with large DNA molecules. Further, these methods can be used to target certain diseases and cell populations by using the targeting characteristics of the carrier.

### a) Nucleic acid based delivery systems

39. Transfer vectors can be any nucleotide construction used to deliver genes into cells (e.g., a plasmid), or as part of a general strategy to deliver genes, e.g., as part of recombinant retrovirus or adenovirus (Ram et al. Cancer Res. 53:83-88, (1993)).

40. As used herein, plasmid or viral vectors are agents that transport the disclosed nucleic acids, such as PTEN-Long into the cell without degradation and include a promoter yielding expression of the gene in the cells into which it is delivered. Viral vectors are, for example, Adenovirus, Adeno-associated virus, Herpes virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors. Retroviruses include Murine Maloney Leukemia virus, MMLV, and retroviruses that express the desirable properties of MMLV as a vector. Retroviral vectors are able to carry a larger genetic payload, i.e., a transgene or marker gene, than other viral vectors, and for this reason are a commonly used vector. However, they are not as useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. A preferred embodiment is a viral vector which has been engineered so as to suppress the immune response of the host organism, elicited by the viral antigens. Preferred vectors of this type will carry coding regions for Interleukin 8 or 10.

41. Viral vectors can have higher transaction (ability to introduce genes) abilities than chemical or physical methods to introduce genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promotor cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in trans.

### (1) Oncolytic vectors

42. In one aspect, the viral vector can be oncolytic. Oncolytic viruses preferentially infect and kill cancer cells either through direct lysis of the infected cells, secretion of a toxin, and/or stimulation of the host anti-tumor immune response. The oncolytic virus can be any type of viral vector including, but not limited to Adenovirus, HSV, Reovirus, Vaccinia virus, VSV, and Measles. For example, HSV based oncolytic viruses can comprise a null mutation or detection of ICP34.5 (also referred to herein as neurovirulence factor gamma-34.5). This mutation results in an HSV vector that is no longer able to replicate in terminally differentiated non-dividing cells, but retains the ability to infect and lyse rapidly dividing cells such as cancers. In one aspect, the viral vector backbone of the disclosed modified viruses expressing PTEN-Long can be an oncolytic HSV vector such as, for example, HSVQuick (HSVQ), HSV1716, HF10, which lacks UL56 protein expression) and OncoVex GM-CSF (which lacks functional expression of ICP34.5 and GM-CSF). Oncolytic viruses can also be oncolytic adenoviruses including, but not limited to, ONYX-015, H101, and KH901; oncolytic reoviruses including, but not limited to, Reolysin and RT3D; oncolytic vaccinia viruses including, but not limited to, GL-ONC1 and JX-594; and oncoclytic measles virus including, but not limited to, MV-NIS. Choice of viral vectors can depend on the tropism of the viral backbone, transgene size, or availability to the scientist. Accordingly, disclosed herein are PTEN-Long expressing HSV viral vectors wherein the viral backbone is an oncolytic HSV-1 backbone that has been modified to target tumor cells and/or to place genes critical to viral replication under the control of a tumor-specific promoter, such as, for example HSVQuick (HSVQ).

### (2) Herpes viral vectors

43. A Herpes virus is an animal virus belonging to the Herpesviridae viral family. Herpes viruses have a wide host range and can accept large transgene payloads. Additionally, Herpes virus have the ability to establish a latent infection and persist indefinitely in a subject.

44. Herpes viral vectors such as Herpes Simplex Virus-1 vectors (for example HSVQuick) can be attenuated to remove genes not essential for replication but affect the host cell range. For example, the virus can be modified to include deletions at neurovirulence factor gamma-34.5 and/or viral ribonucleotide reductase ICP6. Further modification can include deletion or null mutation of ICP47 and/or GM-CSF expression.

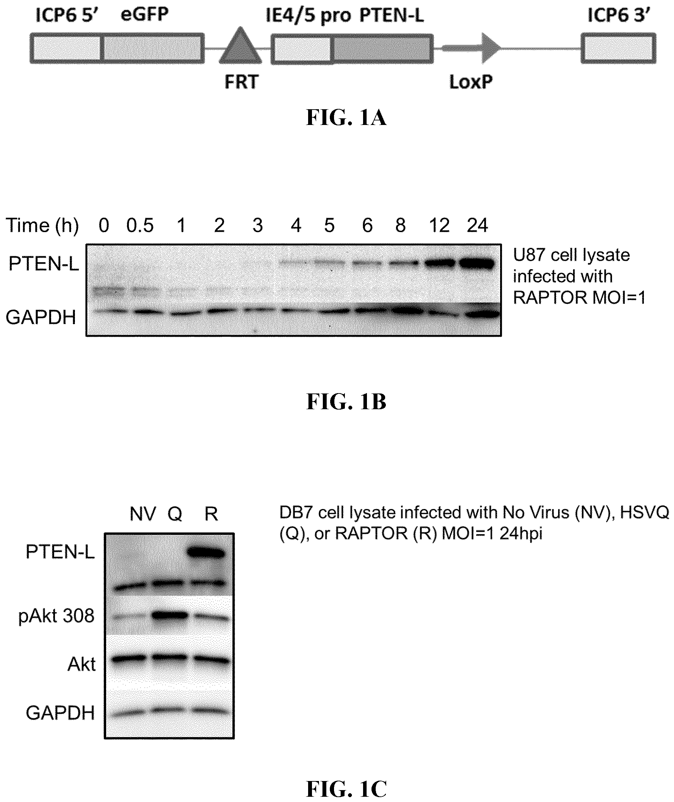

45. The disclosed Herpes viral vectors can also be replication defective. HSV encodes more than 80 viral gene products and about half of these are essential for viral replication. It is relatively easy to make a replication defective mutant by deleting one of the essential genes and providing the gene product in trans by means of a complementing cell line. Such mutant viruses are unable to replicate in non-complementing cells, but they can remain cytotoxic due to the fact that they still express many viral gene products. Viral replication is also readily disrupted by null mutations in immediate early genes that in vitro can be complemented in trans, enabling straightforward production of high-titre pure preparations of non-pathogenic vector. Transduction with replication-defective vectors causes a latent-like infection in both neural and non-neural tissue; the vectors are non-pathogenic, unable to reactivate and persist long-term. For example, the HSVQuick viral vector (HSVQ) was modified to limit infection to non-neuronal tissue. In one aspect, disclosed herein are recombinant viruses comprising a mammalian PTEN-Long gene, wherein the recombinant virus is a herpes virus, such as, for example, a Herpes Simplex virus. In some aspects disclosed herein are PTEN-Long expressing HSV viral vectors wherein the viral backbone is an HSV-1 backbone that has been modified to target tumor cells and/or to place genes critical to viral replication under the control of a tumor-specific promoter, such as, for example HSVQuick (HSVQ). A HSVQ virus expressing a PTEN-Long transgene is referred to herein as a HSV P10 or RAPTOR virus (see, for example, Figure 1A).

### (3) Retroviral Vectors

46. A retrovirus is an animal virus belonging to the virus family of Retroviridae, including any types, subfamilies, genus, or tropisms. Retroviral vectors, in general, are described by Verma, I.M., Retroviral vectors for gene transfer.

47. A retrovirus is essentially a package which has packed into it nucleic acid cargo. The nucleic acid cargo carries with it a packaging signal, which ensures that the replicated daughter molecules will be efficiently packaged within the package coat. In addition to the package signal, there are a number of molecules which are needed in cis, for the replication, and packaging of the replicated virus. Typically a retroviral genome, contains the gag, pol, and env genes which are involved in the making of the protein coat. It is the gag, pol, and env genes which are typically replaced by the foreign DNA that it is to be transferred to the target cell. Retrovirus vectors typically contain a packaging signal for incorporation into the package coat, a sequence which signals the start of the gag transcription unit, elements necessary for reverse transcription, including a primer binding site to bind the tRNA primer of reverse transcription, terminal repeat sequences that guide the switch of RNA strands during DNA synthesis, a purine rich sequence 5' to the 3' LTR that serve as the priming site for the synthesis of the second strand of DNA synthesis, and specific sequences near the ends of the LTRs that enable the insertion of the DNA state of the retrovirus to insert into the host genome. The removal of the gag, pol, and env genes allows for about 8 kb of foreign sequence to be inserted into the viral genome, become reverse transcribed, and upon replication be packaged into a new retroviral particle. This amount of nucleic acid is sufficient for the delivery of a one to many genes depending on the size of each transcript. It is preferable to include either positive or negative selectable markers along with other genes in the insert.

48. Since the replication machinery and packaging proteins in most retroviral vectors have been removed (gag, pol, and env), the vectors are typically generated by placing them into a packaging cell line. A packaging cell line is a cell line which has been transfected or transformed with a retrovirus that contains the replication and packaging machinery, but lacks any packaging signal. When the vector carrying the DNA of choice is transfected into these cell lines, the vector containing the gene of interest is replicated and packaged into new retroviral particles, by the machinery provided in cis by the helper cell. The genomes for the machinery are not packaged because they lack the necessary signals.

### (4) Adenoviral Vectors

49. The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., J. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell, but are unable to form new infectious viral particles. Recombinant adenoviruses have been shown to achieve high efficiency gene transfer after direct, in vivo delivery to airway epithelium, hepatocytes, vascular endothelium, CNS parenchyma and a number of other tissue sites. Recombinant adenoviruses achieve gene transduction by binding to specific cell surface receptors, after which the virus is internalized by receptor-mediated endocytosis, in the same manner as wild type or replication-defective adenovirus.

50. A viral vector can be one based on an adenovirus which has had the E1 gene removed and these virions are generated in a cell line such as the human 293 cell line. In another preferred embodiment both the E1 and E3 genes are removed from the adenovirus genome.

### (5) Adeno-associated viral vectors

51. Another type of viral vector is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An especially preferred embodiment of this type of vector is the P4.1 C vector produced by Avigen, San Francisco, CA, which can contain the herpes simplex virus thymidine kinase gene, HSV-tk, and/or a marker gene, such as the gene encoding the green fluorescent protein, GFP.

52. In another type of AAV virus, the AAV contains a pair of inverted terminal repeats (ITRs) which flank at least one cassette containing a promoter which directs cell-specific expression operably linked to a heterologous gene. Heterologous in this context refers to any nucleotide sequence or gene which is not native to the AAV or B19 parvovirus.

53. Typically the AAV and B19 coding regions have been deleted, resulting in a safe, noncytotoxic vector. The AAV ITRs, or modifications thereof, confer infectivity and site-specific integration, but not cytotoxicity, and the promoter directs cell-specific expression. United States Patent No. 6,261,834 is herein incorporated by reference for material related to the AAV vector.

54. The disclosed vectors thus provide DNA molecules which are capable of integration into a mammalian chromosome without substantial toxicity.

55. The inserted genes in viral and retroviral usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### (6) Large payload viral vectors

56. Molecular genetic experiments with large human herpesviruses have provided a means whereby large heterologous DNA fragments can be cloned, propagated and established in cells permissive for infection with herpesviruses (Sun et al., Nature genetics 8: 33-41, 1994; Cotter and Robertson,. Curr OpinMol Ther 5: 633-644, 1999). These large DNA viruses (herpes simplex virus (HSV) and Epstein-Barr virus (EBV), have the potential to deliver fragments of human heterologous DNA > 150 kb to specific cells. EBV recombinants can maintain large pieces of DNA in the infected B-cells as episomal DNA. Individual clones carried human genomic inserts up to 330 kb appeared genetically stable. The maintenance of these episomes requires a specific EBV nuclear protein, EBNA1, constitutively expressed during infection with EBV. Additionally, these vectors can be used for transfection, where large amounts of protein can be generated transiently in vitro. Herpesvirus amplicon systems are also being used to package pieces of DNA > 220 kb and to infect cells that can stably maintain DNA as episomes.

57. Other useful systems include, for example, replicating and host-restricted non-replicating vaccinia virus vectors.

### b) Non-nucleic acid based systems

58. As disclosed herein, PTEN-Long can be delivered to the target cells in a variety of ways. For example, the compositions can be delivered through electroporation, or through lipofection, or through calcium phosphate precipitation. The delivery mechanism chosen will depend in part on the type of cell targeted and whether the delivery is occurring for example in vivo or in vitro.

59. Thus, the compositions can comprise, in addition to the disclosed PTEN-Long and HSVQ vectors comprising said PTEN-Long transgenes, for example, lipids such as liposomes, such as cationic liposomes (e.g., DOTMA, DOPE, DC-cholesterol) or anionic liposomes. Liposomes can further comprise proteins to facilitate targeting a particular cell, if desired. Administration of a composition comprising a compound and a cationic liposome can be administered to the blood afferent to a target organ or inhaled into the respiratory tract to target cells of the respiratory tract. Regarding liposomes, see, e.g., Brigham et al. Am. J. Resp. Cell. Mol. Biol. 1:95-100 (1989); Felgner et al. Proc. Natl. Acad. Sci USA 84:7413-7417 (1987); U.S. Pat. No.4,897,355. Furthermore, the compound can be administered as a component of a microcapsule that can be targeted to specific cell types, such as macrophages, or where the diffusion of the compound or delivery of the compound from the microcapsule is designed for a specific rate or dosage.

60. In the methods described above which include the administration and uptake of exogenous DNA into the cells of a subject (i.e., gene transduction or transfection), delivery of the compositions to cells can be via a variety of mechanisms. As one example, delivery can be via a liposome, using commercially available liposome preparations such as LIPOFECTIN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI), as well as other liposomes developed according to procedures standard in the art. In addition, the disclosed nucleic acid or vector can be delivered *in vivo* by electroporation, the technology for which is available from Genetronics, Inc. (San Diego, CA) as well as by means of a SONOPORATION machine (ImaRx Pharmaceutical Corp., Tucson, AZ).

61. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). These techniques can be used for a variety of other specific cell types. Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

62. Nucleic acids that are delivered to cells which are to be integrated into the host cell genome, typically contain integration sequences. These sequences are often viral related sequences, particularly when viral based systems are used. These viral intergration systems can also be incorporated into nucleic acids which are to be delivered using a non-nucleic acid based system of deliver, such as a liposome, so that the nucleic acid contained in the delivery system can be come integrated into the host genome.

63. Other general techniques for integration into the host genome include, for example, systems designed to promote homologous recombination with the host genome. These systems typically rely on sequence flanking the nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

### c) In vivo/ex vivo

64. As described above, the compositions can be administered in a pharmaceutically acceptable carrier and can be delivered to the subject=s cells *in vivo* and/or *ex vivo* by a variety of mechanisms well known in the art (e.g., uptake of naked DNA, liposome fusion, intramuscular injection of DNA via a gene gun, endocytosis and the like).

65. If *ex vivo* methods are employed, cells or tissues can be removed and maintained outside the body according to standard protocols well known in the art. The compositions can be introduced into the cells via any gene transfer mechanism, such as, for example, calcium phosphate mediated gene delivery, electroporation, microinjection or proteoliposomes. The transduced cells can then be infused (e.g., in a pharmaceutically acceptable carrier) or homotopically transplanted back into the subject per standard methods for the cell or tissue type. Standard methods are known for transplantation or infusion of various cells into a subject.

### 2. Expression systems

66. The nucleic acids that are delivered to cells typically contain expression controlling systems. For example, the inserted genes in viral and retroviral systems usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### a) Viral Promoters and Enhancers

67. Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a *Hin*dIII E restriction fragment (Greenway, P.J. et al., Gene 18: 355-360 (1982)). Of course, promoters from the host cell or related species also are useful herein.

68. Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers f unction to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, -fetoprotein and insulin), typically one will use an enhancer from a eukaryotic cell virus for general expression. Preferred examples are the SV40 enhancer on the late side of the replication origin (bp 100-270), the cytomegalovirus early promoter enhancer, the polyoma enhancer on the late side of the replication origin, and adenovirus enhancers.

69. The promotor and/or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

70. In certain embodiments the promoter and/or enhancer region can act as a constitutive promoter and/or enhancer to maximize expression of the region of the transcription unit to be transcribed. In certain constructs the promoter and/or enhancer region be active in all eukaryotic cell types, even if it is only expressed in a particular type of cell at a particular time. A preferred promoter of this type is the CMV promoter (650 bases). Other preferred promoters are SV40 promoters, cytomegalovirus (full length promoter), and retroviral vector LTR. In some aspects the constitutive promoter is a promoter that is expressed on the viral backbone such as an immediate early gene promoter for HSV. Accordingly, in one aspect, disclosed herein are recombinant viruses comprising a mammalian PTEN-Long gene, wherein the PTEN-Long gene is operably linked to a constitutive promoter such as, for example, an HSV IE4/5 promoter.

71. In some embodiments the promoter can be a tissue specific or inducible promoter to limit expression to certain tissues or when certain conditions are met or when a supervising physician wants to start and/or stop expression. Inducible promoters can include any inducible promoters known in the art, including, but not limited to Tetracycline-inducible transgenic systems (tetracycline transactivator (tTA) or 'Tet-Off' and reverse tetracycline transactivator (rtTA) or (Tet-On') and Cre-lox. Tissue specific promoters are those promoters where expression is limited to particular tissue/cell types. Tissue specific promoters are well known in the art and can include any tissue specific promoter known or commercially available. Choice of tissue specific promoters is based on the cell-type in which expression is desired. Accordingly, in one aspect, disclosed herein are recombinant viruses comprising a mammalian PTEN-Long gene, wherein the PTEN-Long gene is operably linked to a tissue specific or inducible promoter.

72. It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

73. Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contains a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV40 early polyadenylation signal and consists of about 400 bases. It is also preferred that the transcribed units contain other standard sequences alone or in combination with the above sequences improve expression from, or stability of, the construct.

### b) Markers

74. The viral vectors can include nucleic acid sequence encoding a marker product. This marker product is used to determine if the gene has been delivered to the cell and once delivered is being expressed. Preferred marker genes are the *E. Coli* lacZ gene, which encodes β-galactosidase, and green fluorescent protein.

75. In some embodiments the marker may be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR- cells and mouse LTK- cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

76. The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec. Appl. Genet. 1: 327 (1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

### 3. Sequence similarities

77. It is understood that as discussed herein the use of the terms homology and identity mean the same thing as similarity. Thus, for example, if the use of the word homology is used between two non-natural sequences it is understood that this is not necessarily indicating an evolutionary relationship between these two sequences, but rather is looking at the similarity or relatedness between their nucleic acid sequences. Many of the methods for determining homology between two evolutionarily related molecules are routinely applied to any two or more nucleic acids or proteins for the purpose of measuring sequence similarity regardless of whether they are evolutionarily related or not.

78. It is understood that one way to define any known variants and derivatives or those that might arise, of the disclosed genes and proteins herein is through defining the variants and derivatives in terms of homology to specific known sequences. For example SEQ ID NO: 1 sets forth a particular sequence of a PTEN-Long gene. Specifically disclosed are variants of these and other genes and proteins herein disclosed which have at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 percent homology to the stated sequence. Those of skill in the art readily understand how to determine the homology of two proteins or nucleic acids, such as genes. For example, the homology can be calculated after aligning the two sequences so that the homology is at its highest level.

79. In general, it is understood that one way to define any known variants and derivatives or those that might arise, of the disclosed genes and proteins herein, is through defining the variants and derivatives in terms of homology to specific known sequences. This identity of particular sequences disclosed herein is also discussed elsewhere herein. In general, variants of genes and proteins herein disclosed typically have at least, about 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 percent homology to the stated sequence or the native sequence. Those of skill in the art readily understand how to determine the homology of two proteins or nucleic acids, such as genes. For example, the homology can be calculated after aligning the two sequences so that the homology is at its highest level.

80. Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

81. It is understood that any of the methods typically can be used and that in certain instances the results of these various methods may differ, but the skilled artisan understands if identity is found with at least one of these methods, the sequences would be said to have the stated identity, and be disclosed herein.

82. For example, as used herein, a sequence recited as having a particular percent homology to another sequence refers to sequences that have the recited homology as calculated by any one or more of the calculation methods described above. For example, a first sequence has 80 percent homology, as defined herein, to a second sequence if the first sequence is calculated to have 80 percent homology to the second sequence using the Zuker calculation method even if the first sequence does not have 80 percent homology to the second sequence as calculated by any of the other calculation methods. As another example, a first sequence has 80 percent homology, as defined herein, to a second sequence if the first sequence is calculated to have 80 percent homology to the second sequence using both the Zuker calculation method and the Pearson and Lipman calculation method even if the first sequence does not have 80 percent homology to the second sequence as calculated by the Smith and Waterman calculation method, the Needleman and Wunsch calculation method, the Jaeger calculation methods, or any of the other calculation methods. As yet another example, a first sequence has 80 percent homology, as defined herein, to a second sequence if the first sequence is calculated to have 80 percent homology to the second sequence using each of calculation methods (although, in practice, the different calculation methods will often result in different calculated homology percentages).

### 4. Hybridization/selective hybridization

83. The term hybridization typically means a sequence driven interaction between at least two nucleic acid molecules, such as a primer or a probe and a gene. Sequence driven interaction means an interaction that occurs between two nucleotides or nucleotide analogs or nucleotide derivatives in a nucleotide specific manner. For example, G interacting with C or A interacting with T are sequence driven interactions. Typically sequence driven interactions occur on the Watson-Crick face or Hoogsteen face of the nucleotide. The hybridization of two nucleic acids is affected by a number of conditions and parameters known to those of skill in the art. For example, the salt concentrations, pH, and temperature of the reaction all affect whether two nucleic acid molecules will hybridize.

84. Parameters for selective hybridization between two nucleic acid molecules are well known to those of skill in the art. For example, in some embodiments selective hybridization conditions can be defined as stringent hybridization conditions. For example, stringency of hybridization is controlled by both temperature and salt concentration of either or both of the hybridization and washing steps. For example, the conditions of hybridization to achieve selective hybridization may involve hybridization in high ionic strength solution (6X SSC or 6X SSPE) at a temperature that is about 12-25°C below the Tm (the melting temperature at which half of the molecules dissociate from their hybridization partners) followed by washing at a combination of temperature and salt concentration chosen so that the washing temperature is about 5°C to 20°C below the Tm. The temperature and salt conditions are readily determined empirically in preliminary experiments in which samples of reference DNA immobilized on filters are hybridized to a labeled nucleic acid of interest and then washed under conditions of different stringencies. Hybridization temperatures are typically higher for DNA-RNA and RNA-RNA hybridizations. The conditions can be used as described above to achieve stringency, or as is known in the art. A preferable stringent hybridization condition for a DNA:DNA hybridization can be at about 68°C (in aqueous solution) in 6X SSC or 6X SSPE followed by washing at 68°C. Stringency of hybridization and washing, if desired, can be reduced accordingly as the degree of complementarity desired is decreased, and further, depending upon the G-C or A-T richness of any area wherein variability is searched for. Likewise, stringency of hybridization and washing, if desired, can be increased accordingly as homology desired is increased, and further, depending upon the G-C or A-T richness of any area wherein high homology is desired, all as known in the art.

85. Another way to define selective hybridization is by looking at the amount (percentage) of one of the nucleic acids bound to the other nucleic acid. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the limiting nucleic acid is bound to the non-limiting nucleic acid. Typically, the non-limiting primer is in for example, 10 or 100 or 1000 fold excess. This type of assay can be performed at under conditions where both the limiting and non-limiting primer are for example, 10 fold or 100 fold or 1000 fold below their k_{d}, or where only one of the nucleic acid molecules is 10 fold or 100 fold or 1000 fold or where one or both nucleic acid molecules are above their k_{d}.

86. Another way to define selective hybridization is by looking at the percentage of primer that gets enzymatically manipulated under conditions where hybridization is required to promote the desired enzymatic manipulation. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer is enzymatically manipulated under conditions which promote the enzymatic manipulation, for example if the enzymatic manipulation is DNA extension, then selective hybridization conditions would be when at least about 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer molecules are extended. Preferred conditions also include those suggested by the manufacturer or indicated in the art as being appropriate for the enzyme performing the manipulation.

87. Just as with homology, it is understood that there are a variety of methods herein disclosed for determining the level of hybridization between two nucleic acid molecules. It is understood that these methods and conditions may provide different percentages of hybridization between two nucleic acid molecules, but unless otherwise indicated meeting the parameters of any of the methods would be sufficient. For example if 80% hybridization was required and as long as hybridization occurs within the required parameters in any one of these methods it is considered disclosed herein.

88. It is understood that those of skill in the art understand that if a composition or method meets any one of these criteria for determining hybridization either collectively or singly it is a composition or method that is disclosed herein.

### 5. Nucleic acids

89. There are a variety of molecules disclosed herein that are nucleic acid based, including for example the nucleic acids that encode, for example PTEN-Long, or fragments thereof, as well as various functional nucleic acids. The disclosed nucleic acids are made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if, for example, an antisense molecule is introduced into a cell or cell environment through for example exogenous delivery, it is advantagous that the antisense molecule be made up of nucleotide analogs that reduce the degradation of the antisense molecule in the cellular environment.

### a) Nucleotides and related molecules

90. A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenin-9-yl (A), cytosin-1-yl (C), guanin-9-yl (G), uracil-1-yl (U), and thymin-1-yl (T). The sugar moiety of a nucleotide is a ribose or a deoxyribose. The phosphate moiety of a nucleotide is pentavalent phosphate. An non-limiting example of a nucleotide would be 3'-AMP (3'-adenosine monophosphate) or 5'-GMP (5'-guanosine monophosphate). There are many varieties of these types of molecules available in the art and available herein.

91. A nucleotide analog is a nucleotide which contains some type of modification to either the base, sugar, or phosphate moieties. Modifications to nucleotides are well known in the art and would include for example, 5-methylcytosine (5-me-C), 5-hydroxymethyl cytosine, xanthine, hypoxanthine, and 2-aminoadenine as well as modifications at the sugar or phosphate moieties. There are many varieties of these types of molecules available in the art and available herein.

92. Nucleotide substitutes are molecules having similar functional properties to nucleotides, but which do not contain a phosphate moiety, such as peptide nucleic acid (PNA). Nucleotide substitutes are molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid. There are many varieties of these types of molecules available in the art and available herein.

93. It is also possible to link other types of molecules (conjugates) to nucleotides or nucleotide analogs to enhance for example, cellular uptake. Conjugates can be chemically linked to the nucleotide or nucleotide analogs. Such conjugates include but are not limited to lipid moieties such as a cholesterol moiety. (Letsinger et al., Proc. Natl. Acad. Sci. USA, 1989, 86, 6553-6556). There are many varieties of these types of molecules available in the art and available herein.

94. A Watson-Crick interaction is at least one interaction with the Watson-Crick face of a nucleotide, nucleotide analog, or nucleotide substitute. The Watson-Crick face of a nucleotide, nucleotide analog, or nucleotide substitute includes the C2, N1, and C6 positions of a purine based nucleotide, nucleotide analog, or nucleotide substitute and the C2, N3, C4 positions of a pyrimidine based nucleotide, nucleotide analog, or nucleotide substitute.

95. A Hoogsteen interaction is the interaction that takes place on the Hoogsteen face of a nucleotide or nucleotide analog, which is exposed in the major groove of duplex DNA. The Hoogsteen face includes the N7 position and reactive groups (NH2 or O) at the C6 position of purine nucleotides.

### b) Sequences

96. There are a variety of sequences related to the protein molecules involved in the signaling pathways disclosed herein, for example PTEN-Long, or any of the nucleic acids disclosed herein for making PTEN-Long, all of which are encoded by nucleic acids or are nucleic acids. The sequences for the human analogs of these genes, as well as other anlogs, and alleles of these genes, and splice variants and other types of variants, are available in a variety of protein and gene databases, including Genbank. Those of skill in the art understand how to resolve sequence discrepancies and differences and to adjust the compositions and methods relating to a particular sequence to other related sequences. Primers and/or probes can be designed for any given sequence given the information disclosed herein and known in the art.

### 6. Pharmaceutical carriers/Delivery of pharamceutical products

97. As described above, the compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

98. The compositions may be administered orally, parenterally (e.g., intravenously), by intramuscular injection, by intraperitoneal injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

99. Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795, which is incorporated by reference herein.

100. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

### a) Pharmaceutically Acceptable Carriers

101. The compositions, including antibodies, can be used therapeutically in combination with a pharmaceutically acceptable carrier.

102. Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

103. Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

104. Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

105. The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.

106. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

107. Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

108. Compositions for oral administration include powders or granules, suspensions or solutions in water or non-aqueous media, capsules, sachets, or tablets. Thickeners, flavorings, diluents, emulsifiers, dispersing aids or binders may be desirable..

109. Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### b) Therapeutic Uses

110. Effective dosages and schedules for administering the compositions may be determined empirically, and making such determinations is within the skill in the art. The dosage ranges for the administration of the compositions are those large enough to produce the desired effect in which the symptoms of the disorder are effected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any counterindications. Dosage can vary, and can be administered in one or more dose administrations daily, for one or several days. Guidance can be found in the literature for appropriate dosages for given classes of pharmaceutical products. For example, guidance in selecting appropriate doses for antibodies can be found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical daily dosage of the antibody used alone might range from about 1 µg/kg to up to 100 mg/kg of body weight or more per day, depending on the factors mentioned above.

### C. Methods of treating cancer

111. It is understood and herein contemplated that the disclosed PTEN-Long expressing recombinant viruses are effective in treating cancer. Accordingly, in one aspect, disclosed herein are methods of treating a cancer in a subject comprising administering to the subject any of the PTEN-Long expressing viruses disclosed herein. For example, in one aspect, disclosed herein are methods of treating a cancer in a subject comprising administering to the subject a recombinant virus comprising a mammalian PTEN-Long gene (such as, an oncolytic HSV viral vector comprising a PTEN-Long transgene, for example, HSV-P10).

112. The PTEN-Long expressing viruses disclosed herein can be used to treat any disease where uncontrolled cellular proliferation occurs such as cancers. A non-limiting list of different types of cancers is as follows: lymphomas (Hodgkins and non-Hodgkins), leukemias, carcinomas, carcinomas of solid tissues, squamous cell carcinomas, adenocarcinomas, sarcomas, gliomas, high grade gliomas, blastomas, neuroblastomas, plasmacytomas, histiocytomas, melanomas, adenomas, hypoxic tumours, myelomas, AIDS-related lymphomas or sarcomas, metastatic cancers, or cancers in general.

113. A representative but non-limiting list of cancers that the disclosed PTEN-Long expressing viruses can be used to treat is the following: lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, lung cancers such as small cell lung cancer and non-small cell lung cancer, neuroblastoma, glioblastoma, ovarian cancer, pancreatic cancer, prostate cancer, skin cancer, liver cancer, melanoma, squamous cell carcinomas of the mouth, throat, larynx, and lung, colon cancer, cervical cancer, cervical carcinoma, breast cancer, and epithelial cancer, renal cancer, genitourinary cancer, pulmonary cancer, esophageal carcinoma, head and neck carcinoma, large bowel cancer, hematopoietic cancers; testicular cancer; colon cancer, and rectal cancers.

114. The recombinant viruses expressing mammalian PTEN-Long disclosed herein may also be used for the treatment of precancer conditions such as cervical and anal dysplasias, other dysplasias, severe dysplasias, hyperplasias, atypical hyperplasias, and neoplasias.

115. In one aspect, the disclosed PTEN-Long expressing viruses can be administered therapeutically to inhibit, reduce, or treat a cancer already present in a subject. However, it is also contemplated herein, that the PTEN-Long expressing viruses can be administered prophylactically to inhibit or reduce metastasis. Accordingly, in one aspect disclosed herein are methods of inhibiting or deducing metastasis of a cancer in a subject comprising administering to the subject a recombinant virus expressing a mammalian PTEN-Long gene.

116. As disclosed herein, the ability to treat, inhibit, and/or reduce a cancer can in no small part is the result of an increase in infiltrating immune cells such as NK cells and CD8+ T cells. Typically, CD8 T cells and NK cells would be inhibited from infiltrating the infection site and/or the cancer. Cancer cells often utilize PD1-PD-L1 interaction as a check point to inhibit infiltration of NK cells and CD8 T cells. PD1 is expressed on T cells and when engaged with its ligand PD-L1, a signaling pathway is triggered down PD1 that inhibits proliferation. Cancer cells upregulate PD-L1 and avoid T cell and NK cell mediated death. The disclosed PTEN-Long expressing recombinant viruses inhibit the expression of PD-L1. By inhibiting PD-L1, the PD1-PD-L1 check point is inhibited allowing NK cells and CD8 T cells to infiltrate and kill the cancer. Accordingly, in one aspect, disclosed herein are methods of increasing the number of infiltrating CD8 T cells and/or infiltrating NK cells at the site of a cancer in a subject comprising administering to the subject any of the PTEN-Long expressing recombinant viruses disclosed herein. For example, in one aspect, disclosed herein are methods of increasing the number of infiltrating CD8 T cells and/or infiltrating NK cells at the site of a cancer in a subject comprising administering to the subject a recombinant virus comprising a mammalian PTEN-Long gene (such as, an oncolytic HSV viral vector comprising a PTEN-Long transgene, for example, HSV-P 10).

### D. Examples

117. The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1

118. Oncolytic HSV-1 vectors were generated using the HSVQuik system. This system is uses the G207 viral backbone, which is an F-strain HSV-1 doubly deleted for the neurovirulence factor gamma-34.5 and viral ribonucleotide reductase ICP6 (Fig. 1A). The virus conditionally replicates in tumor cells that are not neuronally derived, are deficient in protein kinase R signaling, and produce their own ribonucleotide reductase during division. The PTEN-L transgene includes an ATG start site 519bp upstream of the canonical PTEN start site (SEQ ID NO: 1). This second start site was mutated in the transgene to exclude canonical PTEN by mutating its second ATG start site to ATA using site-directed mutagenesis. This mutation forces expression of the full PTEN-L gene, and prevent the expression of the short, intracellular form of PTEN. The mutated PTEN-L transgene was cloned into the G207 viral backbone following the HSVquik protocol.

119. HSV-P10 was amplified and purified using a standard sucrose purification protocol. 10-20 150mm plates of 70% confluent Vero cells were infected at low MOI in a minimal volume of 2%-FBS DMEM. Infected plates were transferred to 34C in a humidified incubator with 5% C0₂ + air. Once the majority of the cells on the plate started rounding (usually 3-4 days), cells and media were scraped and collected. Cells pelleted by centrifugation and supernatants transferred to separate 50ml conical tubes. All cells were resuspended together in 20-30ml of supernatant and cells and media were flash frozen in liquid nitrogen and stored at - 80C. The frozen cells were thawed in a 37C water bath to 4C and were sonicated to release viral particles from intact cells. Cell debris was again pelleted by centrifugation and media containing virus particles was combined with the separated media containing no cells. Viral particles were pelleted by ultra-centrifugation and resuspended in sterile saline. Resuspended virus was overlaid on top of a 30% sucrose layer, and the separated layers were again ultra-centrifuged to purify only virus particles. Pelleted virus was resuspended in a minimal volume of 20% glycerol in sterile saline and aliquoted in 20u1 aliquots to be stored at -80C.

120. To test in vitro and in vivo efficacy of a PTEN-L expressing virus (HSV-P10 also referred to as RAPTOR), U87 glioblastoma cells were infected with HSV-P10 (i.e., RAPTOR) at a multiplicity of infection of 1 and the expression of PTEN-Long was measured at various time points (Figure 1B). PTEN-Long expression is observed within 4 hours post infection. Next, to compare the effect of PTEN-Long on the phosphorylation of AKT, DB7 cells were infected with cell lysate from uninfected cells (NV) or cells infected with HSVQ vector alone (Q) or HSV-P10 (i.e., PTEN-Long expressing HSVQ) (R). Here DB7 cells infected with HSVQ-P10 expressed PTEN-Long and reduced AKT phosphorylation. Compared to HSVQ infected cells.

121. PTEN is commonly lost in Breast cancer brain metastasis (BCBM). To confirm this shows representative hematoxylin and eosin (H&E) and PTEN staining from a sectioned human breast cancer brain metastasis (top, 40X) and FVB/N mouse bearing intracranial DB7 (murine breast cancer cells) (bottom, 20X). The staining confirms significant loss of PTEN in BCBM cells.

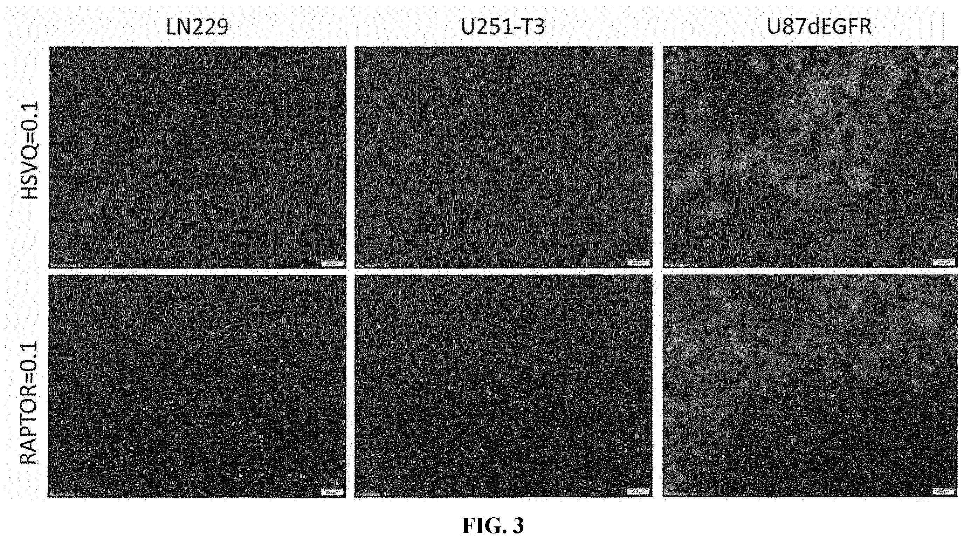

122. To show that RAPTOR virus (i.e., HSV-P10) has the same cellular tropism and proliferative ability as HSVQ virus, LN229 (glioblastoma cell line), U251-T3 (glioma cells), and U87ΔEGFR (glioma celld) cells were infected with either HSVQ or HSV-P10 at an multiplicity of infection (MOI) of 0.1. Cells were observed for viability and infection. As shown in Figure 3 both HSVQ and HSV-P10 infected all three cell types and were able to completely lyse LN229 and U251-T3 cells.

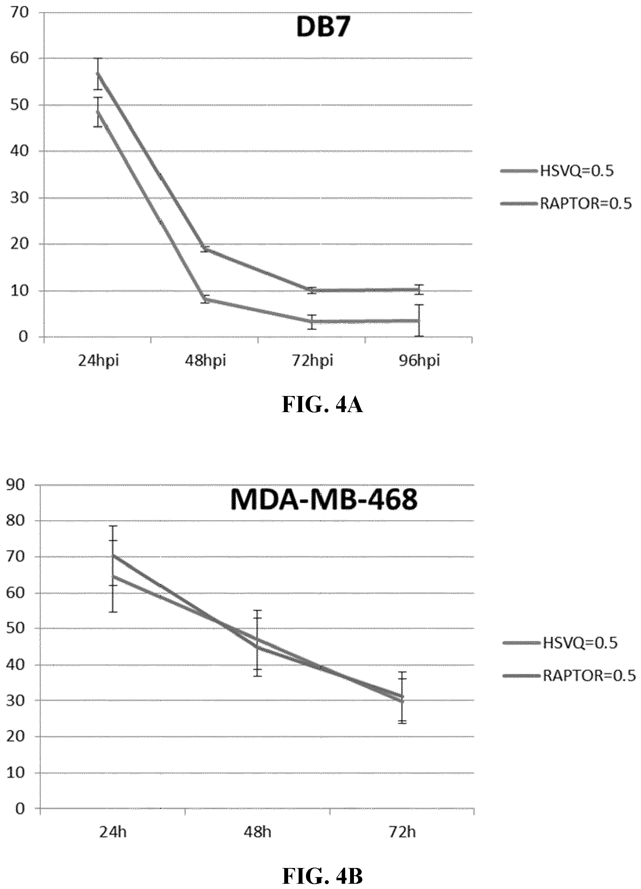

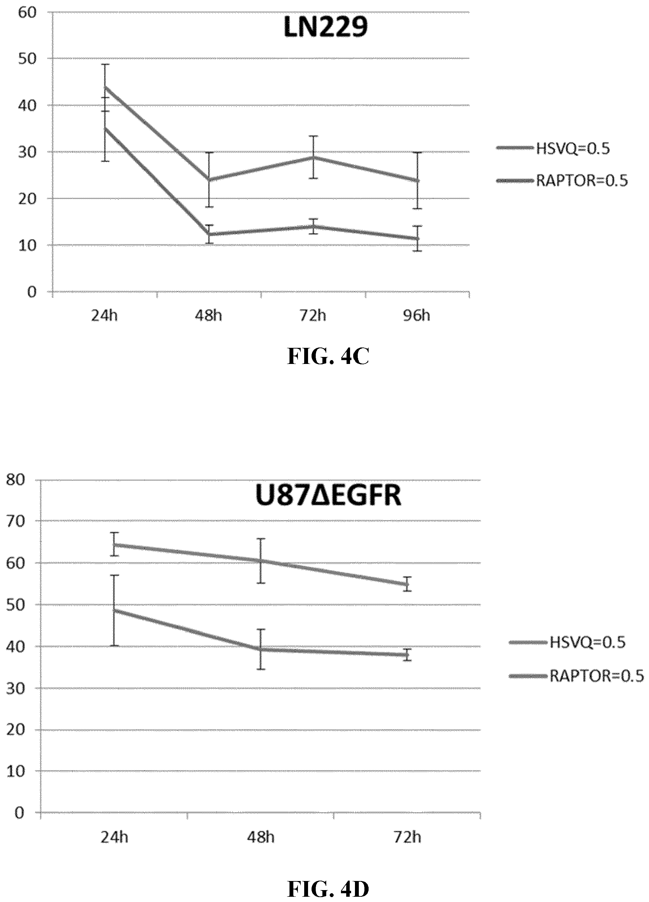

123. Next, MTT assays were performed on DB7 (Figure 4A), MDA-MB-468 (human breast cancer)(Figure 4B), LN229 (Figure 4C) and U87ΔEGFR cells (Figure 4D) and it was observed that PTEN-Long expression does not inhibit HSV-P10 cell killing in multiple cell lines. In each cell scenario the rate of lysis of infected cells was identical. Differences in magnitude were determined to be due to variance in viral burden administered to the cells rather than a true difference in the virus infections as evidenced by any difference being perfectly maintained over the 96 hours of culture.

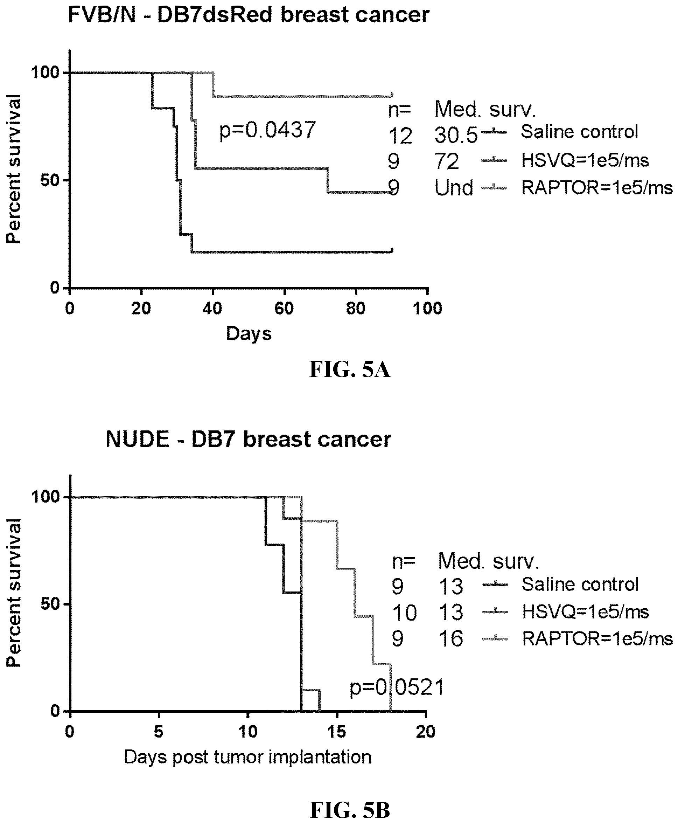

124. To see how the expression of PTEN-Long effected in vivo tumor burden, DB7-dsRed tumor bearing FVB/N mice immune competent and DB7 bearing nude mice were infected with a saline control or HSVQ viral backbone or PTEN-Long expressing HSV-P10 (i.e., RAPTOR)(Figure 5). BY 40days post infection 90% of mock infected animals and 50% HSVQ infected animals and died. By contrast at 100 days post infection, over 90% of HSV-P10 animals had survived. In immunocompromised nude mice, all tumor bearing mice were dead by 15 days in the HSVQ and saline groups and by day 19 in the RAPTOR infected group. This shows that HSV-P10 enhances survival in mice bearing breast cancer brain metastases.

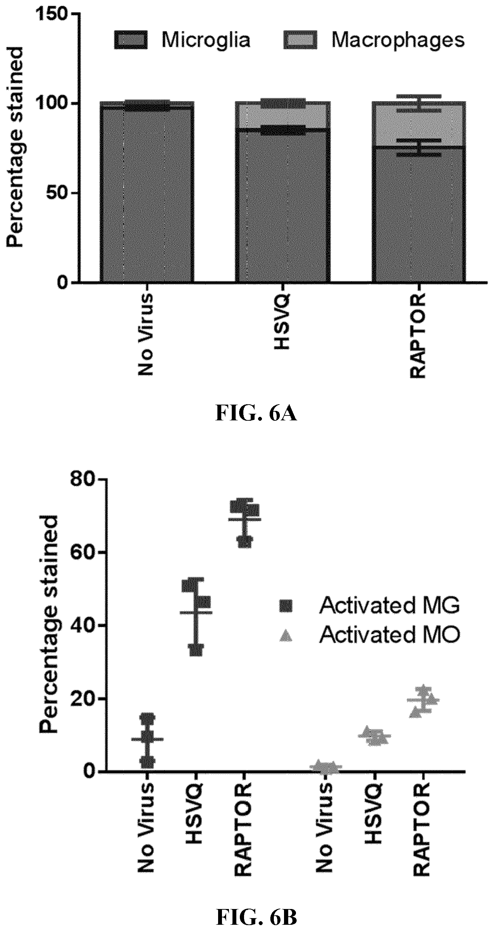

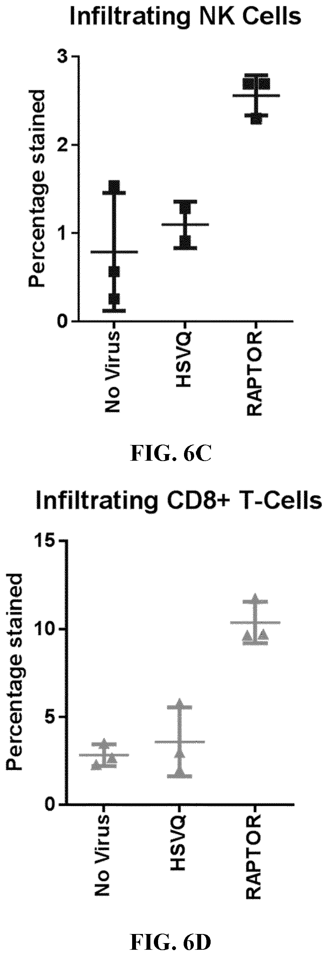

125. To determine the mechanism of protection in HSV-P10 infected mice, infected tumor cells were removed and measured for the presence of infiltrating macrophage, NK cells, and CD8 T cells (Figure 6). Staining for CD11b+ and F4/80+ it was observed that the ratio of CD11b⁺ F4/80⁺ microglia (CD45^{lo-int}) to macrophages (CD45^{hi}) decreased in HSVQ and HSV-P10 cells with relatively more macrophages being present in the HSV-P10 infected cells (Figure 6A). When measured to determine the activation state of the microglia and macrophage, it was further observed that the percentage of activated cells also increased in HSVQ and ISV-P10 infected cells with significantly more activation in the HSV-P10 population (Figure 6B). Measuring for infiltrating NK cells (Figure 6C) and infiltrating CD8 T cells (Figure 6D) showed that while HSVQ did not have a statistical increase in the percentage of infiltrating NK cells or CD8 T cells, RAPTOR infected cells (i.e., HSV-P10 infected cells) showed significant increase in the percentage of NK cells and CD8 T cells relative to HSVQ and mock infected.

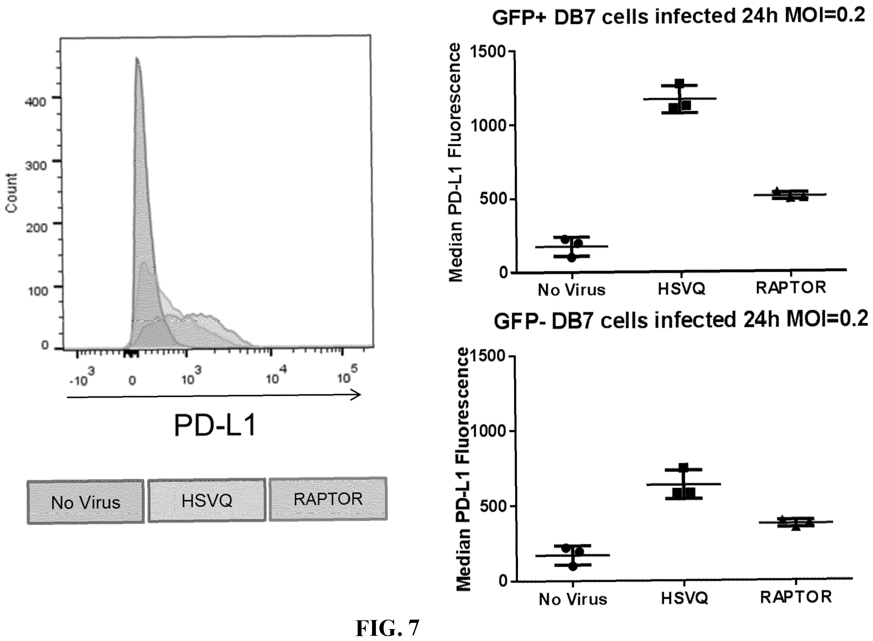

126. Postulating that the increase in infiltrating NK cells and infiltrating CD8 T cells could be due to a reduction in PD-L1 expression which would normally function to signal through PD1 to inhibit NK cell and CD8 T cell proliferation, cells were tested for PD-L1 expression (Figure 7). DB7 cells that were mock infected showed almost undetectable levels of PD-L1 which would be expected. Similarly expected, HSVQ infected cells showed virally induced PD-L1 expression 24 hours post infection. However, cells infected with the PTEN-Long expressing HSV-P10 (i.e., RAPTOR) showed significant reduction in PD-L1 expression. AS the only difference between HSVQ backbone and HSV-P10 is the PTEN-Long transgene, this reduction in PD-L1 is a direct result of PTEN-Long expression.

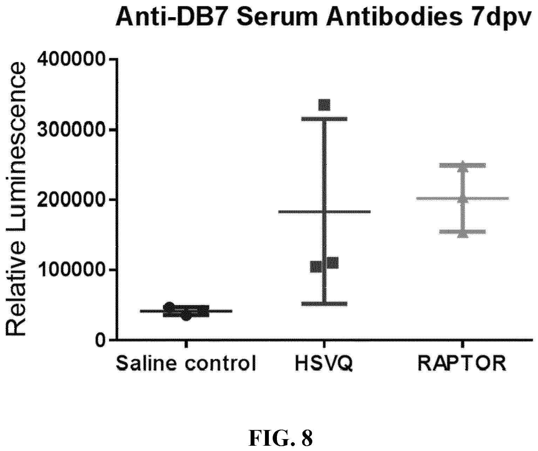

127. Additionally, HSV-P10 infected animals were able to generate anti-DB7 antibodies (Figure 8).

### CONCLUSIONS

128. HSV-P10 virus is a Herpes Simplex Virus type 1 with doubly deleted gamma-34.5 genes and an insertional mutation in viral ICP6, including GFP and PTEN-Long expression vectors. The HSV-P10: expresses PTEN-L in and secretes PTEN-L from infected cells and also reduces Akt phosphorylation in infected cells. HSV-P10 shows equivalent replication in glioma cells compared with HSVQ and kills glioma and breast cancer cell lines as effectively as HSVQ.

129. However, HSV-P10 enhances survival in immune competent mice bearing DB7 breast cancer brain metastases. This is partially the result on an increase in recruitment of activated immune effector cells in DB7 tumor-bearing brain hemispheres which are no longer being suppressed by expression of PD-L1 as it is shown herein that HSV-P10 reduces PD-L1 expression in infected and neighboring uninfected cells. Additionally, HSV-P10 induces robust antigen spreading against DB7 cancer antigens

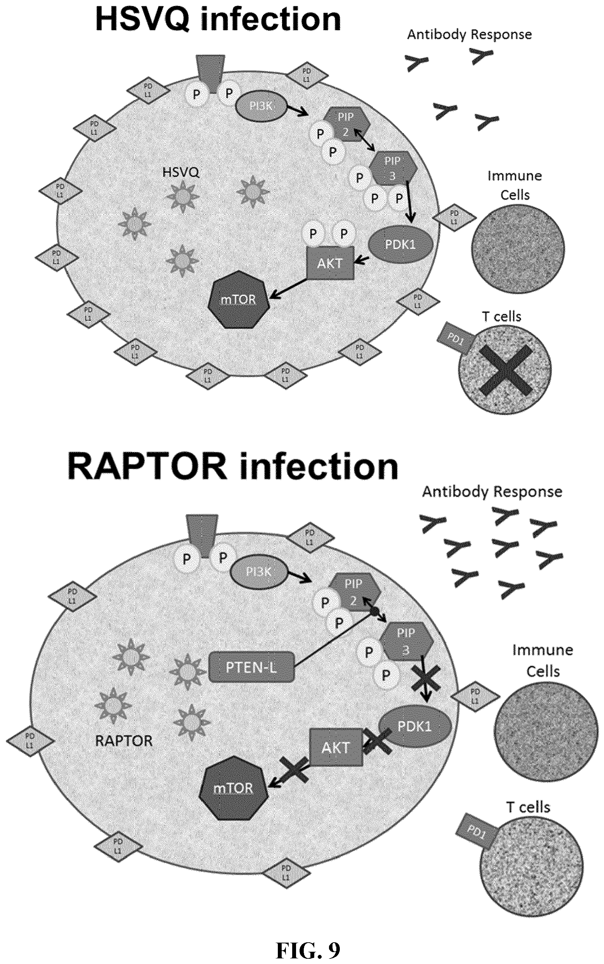

130. HSV-P10 acts in 3 major ways to elicit tumor cell killing: First, the virus is able to selectively replicate in and destroy infected tumor cells. Second, HSV-P10 induces secretion of the tumor suppressor PTEN-Long, which can act to locally slow the growth of uninfected tumor cells, and can also act as a Damage Associated Molecular Pattern molecule to recruit the innate immune system to the site of infection. Third, HSV-P10 acts as a "cancer vaccine", provoking an adaptive immune response against the tumor cells by exposing non-self tumor peptides to the immune system through tumor cell lysis and immune cell recruitment to the site of infection (Figure 9).

The following alphabetised list of embodiments is disclosed:
A) A recombinant virus comprising a mammalian PTEN-Long gene.
B) The recombinant virus of embodiment A, wherein the virus has been modified to target tumor cells.
C) The recombinant virus of embodiment A or B, wherein the virus has been modified to place genes critical to viral replication under the control of a tumor-specific promoter.
D) The recombinant virus of embodiments A-C, wherein the recombinant virus comprises a Herpes Simplex Virus- 1 backbone.
E) The recombinant virus of embodiments A-D, wherein the PTEN-Long gene is operatively linked to a tissue specific promoter.
F) The recombinant virus of embodiments A-D, wherein the PTEN-Long gene is operatively linked to an inducible promoter.
G) The recombinant virus of embodiments A-D, wherein the PTEN-Long gene is operatively linked to a constitutive promoter.
H) A method of treating a cancer in a subject comprising administering to the subject the virus of embodiments A-G.
I) A method of inhibiting PD-L1 in a cancer cell comprising administering to the subject the virus of embodiments A-G.
J) A method of increasing the number of infiltrating CD8 T cells at the site of a cancer in a subject, comprising administering to the subject the virus of embodiments A-G.
K) A method of increasing the number of infiltrating Natural Killer cells at the site of a cancer in a subject, comprising administering to the subject the virus of embodiments A-G.
L) A method of inhibiting the metastasis of a cancer in a subject comprising administering to the subject the virus of embodiments A-G.
M) A method of treating a cancer in a subject comprising administering to the subject a recombinant virus comprising a mammalian PTEN-Long gene.
N) The method of embodiment M, wherein the virus has been modified to target tumor cells.
O) The method of embodiment M, wherein the recombinant virus comprises a Herpes Simplex Virus-1 backbone.

### E. References

1 Cerami, E., Demir, E., Schultz, N., Taylor, B. S., & Sander, C. (2010). Automated network analysis identifies core pathways in glioblastoma. PloS One, 5(2), e8918.
2 Hopkins, B. D., Fine, B., Steinbach, N., Dendy, M., Rapp, Z., Shaw, J., Parsons, R. (2013). A secreted PTEN phosphatase that enters cells to alter signaling and survival. Science (New York, N.Y.), 341(6144), 399-402.
3 Mineharu Y, Kamran N, Lowenstein PR, Castro MG. Blockade of mTOR signaling via rapamycin combined with immunotherapy augments antiglioma cytotoxic and memory T-cell functions. Mol Cancer Ther. 2014; 13(12):3024-3036.
4 Terada, K., Wakimoto, H., Tyminski, E., Chiocca, E. A., & Saeki, Y. (2006). Development of a rapid method to generate multiple oncolytic HSV vectors and their in vivo evaluation using syngeneic mouse tumor models. Gene Therapy, 13(8), 705-14.
5 Zhang L, Zhang S, Yao J, et al. Microenvironment-induced PTEN loss by exosomal microRNA primes brain metastasis outgrowth. Nature. 2015;527(7576): 100-104.

### F. Sequences

**SEQ ID NO: 1 PTEN-Long coding region**

ATGGAGCGGGGAGGAGAAGCGGCGGCGGCGGCCGCGGCGGCTGCAGCTCCAG
GGAGGGGGTCTGAGTCGCCTGTCACCATTTCCAGGGCTGGGAACGCCGGAGAGTTG
GTCTCTCCCCTTCTACTGCCTCCAACACGGCGGCGGCGGCGGCACATCCAGGG
ACCCGGGCCGGTTTTAAACCTCCCGTCCGCCGCCGCCGCACCCCCCGTGGCCCGGGC
TCCGGAGGCCGCCGGCGGAGGCAGCCGTTCGGAGGATTATTCGTCTTCTCCCCATTC
CGCTGCCGCCGCTGCCAGGCCTCTGGCTGCTGAGGAGAAGCAGGCCCAGTCGCTGC
AACCATCCAGCAGCCGCCGCAGCAGCCATTACCCGGCTGCGGTCCAGAGCCAAGCG
GCGGCAGAGCGAGGGGCATCAGCTACCGCCAAGTCCAGAGCCATTTCCATCCTGCA
GAAGAAGCCCCGCCACCAGCAGCTTCTGCCATCTCTCTCCTCCTTTTTCTTCAGCCAC
AGGCTCCCAGACATAACAGCCATCATCAAAGAGATCGTTAGCAGAAACAAAAGGA
GATATCAAGAGGATGGATTCGACTTAGACTTGACCTATATTTATCCAAACATTATTG
CTATGGGATTTCCTGCAGAAAGACTTGAAGGCGTATACAGGAACAATATTGATGAT
GTAGTAAGGTTTTTGGATTCAAAGCATAAAAACCATTACAAGATATACAATCTTTGT
GCTGAAAGACATTATGACACCGCCAAATTTAATTGCAGAGTTGCACAATATCCTTTT
GAAGACCATAACCCACCACAGCTAGAACTTATCAAACCCTTTTGTGAAGATCTTGAC
CAATGGCTAAGTGAAGATGACAATCATGTTGCAGCAATTCACTGTAAAGCTGGAAA
GGGACGAACTGGTGTAATGATATGTGCATATTTATTACATCGGGGCAAATTTTTAAA
GGCACAAGAGGCCCTAGATTTCTATGGGGAAGTAAGGACCAGAGACAAAAAGGGA
GTAACTATTCCCAGTCAGAGGCGCTATGTGTATTATTATAGCTACCTGTTAAAGAAT
CATCTGGATTATAGACCAGTGGCACTGTTGTTTCACAAGATGATGTTTGAAACTATT
CCAATGTTCAGTGGCGGAACTTGCAATCCTCAGTTTGTGGTCTGCCAGCTAAAGGTG
AAGATATATTCCTCCAATTCAGGACCCACACGACGGGAAGACAAGTTCATGTACTTT
GAGTTCCCTCAGCCGTTACCTGTGTGTGGTGATATCAAAGTAGAGTTCTTCCACAAA
CAGAACAAGATGCTAAAAAAGGACAAAATGTTTCACTTTTGGGTAAATACATTCTT
CATACCAGGACCAGAGGAAACCTCAGAAAAAGTAGAAAATGGAAGTCTATGTGAT
CAAGAAATCGATAGCATTTGCAGTATAGAGCGTGCAGATAATGACAAGGAATATCT
AGTACTTACTTTAACAAAAAATGATCTTGACAAAGCAAATAAAGACAAAGCCAACC
GATACTTTTCTCCAAATTTTAAGGTGAAGCTGTACTTCACAAAAACAGTAGAGGAGC
CGTCAAATCCAGAGGCTAGCAGTTCAACTTCTGTAACACCAGATGTTAGTGACAAT
GAACCTGATCATTATAGATATTCTGACACCACTGACTCTGATCCAGAGAATGAACCT
TTTGATGAAGATCAGCATACACAAATTACAAAAGTCTGA

## Claims

1. A recombinant oncolytic virus comprising a gene encoding a mammalian PTEN-Long or a fragment thereof comprising PTEN.

2. The recombinant oncolytic virus of claim 1, wherein the virus has been modified to target tumor cells.

3. The recombinant oncolytic virus of claim 1 or claim 2, wherein the virus has been modified to place genes critical to viral replication under the control of a tumor-specific promoter.

4. The recombinant oncolytic virus of any one of claims 1 to 3, wherein the gene is operatively linked to a tissue specific promoter.

5. The recombinant oncolytic virus of any one of claims 1 to 4, wherein the gene is operatively linked to an inducible promoter.

6. The recombinant oncolytic virus of claim 1 or claim 2, wherein the gene is operatively linked to a constitutive promoter.

7. The recombinant oncolytic virus of any one of claims 1 to 6, wherein the recombinant virus comprises a Herpes Simplex Virus-1 backbone.

8. The recombinant oncolytic virus of any one of claims 4 to 7, wherein the recombinant oncolytic virus is HSVQuik.

9. The recombinant oncolytic virus of any one of claims 1 to 8, comprising a gene encoding a mammalian PTEN-Long.

10. The recombinant oncolytic virus of any one of claims 1 to 9, wherein the fragment of PTEN-Long consists of the amino acid sequence of PTEN.

11. The recombinant oncolytic virus of any one of claims 1 to 10 for use in a method of treating a cancer in a subject.

12. The recombinant oncolytic virus of any one of claims 1 to 10 for use in a method of inhibiting PD-L1 in a cancer cell in a subject.

13. The recombinant oncolytic virus of any one of claims 1 to 10 for use in a method of increasing the number of infiltrating CD8 T or Natural Killer cells at the site of a cancer in a subj ect.

14. The recombinant oncolytic virus of any one of claims 1 to 10 for use in a method of inhibiting the metastasis of a cancer in a subject.

15. An *ex vivo* method of inhibiting PD-L1 in a cancer cell comprising contacting the cancer cell with the recombinant oncolytic virus of any one of claims 1 to 10.
